# EUROPÄISCHE PATENTSCHRIFT

(11) **EP 2 421 427 B1**
(45) Veröffentlichungstag und Bekanntmachung des Hinweises auf die Patenterteilung: **28.12.2016**
(21) Anmeldenummer: 10713848.9
(22) Anmeldetag: 13.04.2010
(51) Int. Cl.: A61B 1/247, A61B 1/045, A61B 1/00, G02B 23/02, G02B 3/14, G02B 7/09, G02B 7/08

(54) **DENTALE ODER MEDIZINISCHE KAMERA**
DENTAL OR MEDICAL CAMERA
CAMÉRA DENTAIRE OU MÉDICALE

(30) Priorität: 20.04.2009 DE 102009017819
(43) Veröffentlichungstag der Anmeldung: 29.02.2012
(73) Patentinhaber: Dürr Dental AG, 74321 Bietigheim-Bissingen (DE)
(72) Erfinder: MAIER, Raimund, 71732 Tamm (DE); GEBHARDT, Herbert, 74936 Siegelsbach (DE)
(74) Vertreter: Ostertag, Reinhard
(86) Internationale Anmeldenummer: PCT/EP2010/002254
(87) Internationale Veröffentlichungsnummer: WO 2010/121723

(56) Entgegenhaltungen:
- EP-A1- 1 780 575
- EP-A1- 1 847 212
- WO-A1-99/06871
- WO-A1-03/101089
- DE-A1-102004 001 856
- US-A- 4 389 565
- US-A1- 2005 020 883

## Beschreibung

Die Erfindung betrifft eine dentale oder medizinische Kamera mit einer Mehrzahl von Komponenten, darunter eine Optik und ein Bildwandler, welche mindestens ein einstellbares Element umfassen.

Ein einstellbares Element einer solchen Kamera, das über ein mechanisches Stellmittel wie beispielsweise einen Drehring oder einen Schieber eingestellt wird, ermöglicht, dem Bediener, die Abbildungseigenschaften der Kamera zu beeinflussen. Für eine möglichst einfache Bedienung der Kamera wäre jedoch eine vereinfachte Einstellbarkeit der Kamera wünschenswert.

Eine Vorrichtung gemäß dem Oberbegriff des unabhängigen Anspruchs 1 ist aus der Druckschrift EP 1 847 212 A1 bekannt.

Aufgabe der Erfindung ist es, eine dentale oder medizinische Kamera anzugeben, deren Handhabung vereinfacht ist.

Erfingdungsgemäß wird dies durch eine dentale oder medizinische Kamera gemäß dem Oberbegriff des Anspruchs 1 erreicht, bei der für mindestens eines der einstellbaren Elemente mindestens eine Servoeinstelleinrichtung vorgesehen ist.

Mittels einer Servoeinstelleinrichtung, die entsprechend einem anliegenden elektrischen Signal ein einstellbares Element der Kamera ansteuert, wird eine elektrische Einstellbarkeit der Kamera erreicht und die Handhabbarkeit der Kamera vereinfacht, da man die eigentlichen Einstellmittel frei von Rücksichtnahmen auf mechanische Erfordernisse wählen kann.

So können Kameraparameter, wie Abbildungseigenschaften, zu denen beispielsweise die Beleuchtung, der Maßstab, Tiefenschärfe, die Lichtmenge oder der Fokus zählen, oder Bildeigenschaften, zu denen beispielsweise der Kontrast, der Farb- oder Weißabgleich sowie Bildauswertungen oder Bildbearbeitungen zählern, verändert werden.

Ein einstellbares Element kann beispielsweise eine bewegliche Halterung einer Linse der Optik sein, durch deren Verschiebung der Fokus, die Lage der Bildebene und/oder die Brennweite der Kamera verändert werden kann. Ein weiteres wichtiges einstellbares Element kann eine Blende sein, die über eine elektromechanische Servoeinstelleinrichtung eingestellt wird.

Auch elektrisch-elektrisch arbeitende Servoeinstelleinrichtungen wie beispielsweise eine spannungsgesteuerte Flüssiglinse oder ein Programm-Modul (z.B. für Veränderungen der Auswertung des Bildes in einer Steuereinheit, mit dem Eigenschaften wie Kontrast oder Helligkeit des Bildes beeinflussbar sind) sind denkbar. Die einstellbaren Elemente können auch eine Beleuchtungseinrichtung umfassen, welche entsprechend einer gewünschten Ausleuchtung des abzubildenden Objekts angesteuert wird.

Erfindungsgemäss ist vorgesehen, dass die Kamera mindestens einen Geber aufweist, durch den mindestens zwei verschiedene Betriebsarten der Kamera unterscheidbar sind und dessen Ausgangssignal bei der Ansteuerung mindestens einer der Servoeinstelleinrichtungen verwendet wird.

Eine dentale Kamera wird z.B. häufig in zwei unterschiedlichen Betriebsarten verwendet. In einer intraoralen Betriebsart wird die dentale Kamera in den Mundraum eingeführt, um Details des Mundinneren beispielsweise zu behandelnde Zähne abzubilden. Bei einer extraoralen Betriebsart wird die dentale Kamera dazu verwendet, die Frontpartie des Gebisses von außerhalb des Mundraums abzubilden.

Durch Geber, die zwischen verschiedenen Betriebsarten unterscheiden können und deren Ausgangssignale bei der Ansteuerung der Servoeinstelleinrichtungen verwendet werden, können eventuell notwendige Anpassungen der Abbildungseigenschaften an die jeweilige Betriebsart automatisch vorgenommen werden.

Falls nur zwischen zwei Betriebsarten unterschieden werden muss, kann bereits ein Geber ausreichen, bei dessen Ansprechen gemäß der einen der beiden Betriebsarten gearbeitet wird während die Vorgaben für die andere bei Nicht-Ansprechen des Gebers eingestellt verwendet werden. Aus Redundanzgründen und zur eindeutigen Identifikation der gerade gewünschten Betriebsart können auch mehrere Geber verwendet werden, die vorzugsweise unterschiedliche Größen erfassen oder die gleiche Größe nach unterschiedlichen Prinzipien erfassen.

Bei einer Weiterbildung der Erfindung ist vorgesehen, dass unter den Gebern ein Abstandssensor, vorzugsweise ein optischer Abstandssensor, ist.

Zum einen kann mit einem Abstandssensor beispielsweise der Abstand zu den Zähnen gemessen werden und so bei festgelegten Abständen zwischen einer intraoralen und einer extraoralen Betriebsart unterschieden und dementsprechend zwischen unterschiedlichen Arbeitsweisen der Kamera umgeschaltet werden. Oder es können einzelne Funktionen ein- oder abgeschaltet werden. Zum anderen können Abbildungseigenschaften der Kamera, wie insbesondere der Fokus, innerhalb einer Betriebsart an die gemessenen Abstände angepasst werden. Vorzugsweise ist der Abstandssensor ein optischer Abstandssensor, beispielsweise ein Reflexkoppler, welcher IR-Licht aussendet, das an dem abzubildenden Objekt reflektiert wird und mit einem Fototransistor ausgewertet wird.

Ein Bewegungs- und ein Abstandssensor können auch in Form einer digitalen Auswertung realisiert werden, die bestimmte Eigenschaften des von dem Bildwandlers aufgenommenen Bildes als Anzeichen für Bewegung oder als Maß für den Abstand heranzieht. Die Ausgangssignale des Sensors werden dann zumindest mittelbar zur Steuerung der Servoeinstelleinrichtungen zur Veränderung der Abbildungseigenschaften, wie Fokus und Blende, und/oder der Bildeigenschaften, wie Kontrast, Farbwert und/oder Weißabgleich, verwendet.

Bei einer Weiterbildung der Erfindung ist vorgesehen, dass der Bewegungs- oder Abstandssensor nahe einem Eintrittsfenster der Kamera angeordnet ist.

Damit wird gewährleistet, dass der Sensor das tatsächlich abzubildende Objekt erfasst.

Bei einer Weiterbildung der Erfindung ist vorgesehen, dass unter den Gebern ein Lagesensor ist.

Auch mittels eines Lagesensors, welcher einen Neigungssensor oder eine Neigungsschaltereinheit umfassen kann, oder mittels eines Vibrationssensors kann zwischen unterschiedlichen Betriebsarten unterschieden werden. So kann beispielsweise ein Neigungssensor zwischen einem vorwiegend waagrechten Halten der Kamera bei einer intraoralen Betriebsart und einem überwiegend senkrechten Halten der Kamera in einer extraoralen Betriebsart unterscheiden. Mit Hilfe von Lagesensoren kann auch erkannt werden, ob die Kamera gerade vom Bediener geführt wird oder ob sie sich gerade in einer Ablage befindet, die zum Bereithalten der Kamera an einem Behandlungsplatz vorgesehen sein kann. Dabei können sowohl aufwendigere Beschleunigungssensoren und Lagesensoren zur exakten Koordinatenbestimmung als auch einfache Lagesensoren, wie einfache Positionsschalter, mit denen nur zwei unterschiedliche Lagen erkannt werden können, verwendet werden.

Bei einer Weiterbildung der Erfindung ist vorgesehen, dass unter den Gebern ein manuell betätigter Geber ist.

Ein manuell betätigter Geber ermöglicht dem Bediener zwischen unterschiedlichen Betriebsarten umzuschalten. Die Betätigung der Geber kann dabei auch für den Bediener unbewusst erfolgen. Beispielsweise erfordern verschiedene Betriebsarten meist auch ein Fassen der Kamera durch den Bediener an unterschiedlichen Stellen, sodass besonders vorteilhaft einige der manuell betätigten Geber an unterschiedlichen Stellen der Kamera angeordnet sind.

Bei einer Weiterbildung der Erfindung ist vorgesehen, dass der manuell betätigte Geber ein kapazitiver Sensor, ein optischer Sensor oder ein Drucksensor ist.

Ein kapazitiver Sensor hat den Vorteil, dass keine beweglichen, mechanischen Teile notwendig sind, die das flüssigkeitsdichte Gehäuse durchdringen. Auch ein optischer Sensor, der hinter einem transparenten Fenster angeordnet sein kann, ermöglicht ein einfach abzudichtendes Gehäuse der Kamera (z.B. ein einstückig gespritztes Gehäuse aus transparentem Material, welches ggf. lokal bedruckt wird), was im Hinblick auf eine einfache und effektive Reinigung der Kamera vorteilhaft ist. Eine andere Möglichkeit eines manuell betätigten Gebers ist ein elastomeres Geberteil mit einem Dehnungsmessstreifen, das in einem Bereich angeordnet ist, in dem die Kamera vom Bediener erfasst wird. Durch Druck auf das Geberteil wird dieses verformt und der Dehnungsmessstreifen spricht an.

Bei einer Weiterbildung der Erfindung ist vorgesehen, dass mindestens einer der Geber Teil einer Eingabeeinheit ist, z.B. einer Handeingabeeinheit, einer Fußeingabeeinheit oder eines Bedienpanels einer Kamerabetriebseinheit.

Dadurch, dass die Geber Teil einer Eingabeeinheit sind, wird der Aufbau der Kamera vereinfacht und der Bediener kann diese verwenden, ohne Gefahr zu laufen, unbeabsichtigt eine Veränderung der Betriebsarten und damit einhergehend der Kameraparameter hervorzurufen. Fußeingabeeinheiten sind als Eingabeeinheit besonders vorteilhaft, da dem Bediener so beide Hände zur Platzierung und Handhabung der Kamera zur Verfügung stehen.

Bei einer Weiterbildung der Erfindung ist vorgesehen, dass unter den Gebern eine Sprachsteuerung ist.

Bei der Verwendung einer Sprachsteuerung können Änderungen der Kameraparameter durch einfache Sprachbefehle erreicht werden, ohne dass der Bediener eine einmal eingenommene Position bezüglich des Patienten verändern muss.

Erfindungsgemäss ist vorgesehen, dass die Kamera ein Zeitglied umfasst, welches während eines festgelegten Zeitraums einen Wechsel der Betriebsart verhindert oder eine vorgegebene Betriebsart einstellt.

Dadurch, dass ein Wechsel der Betriebsart für bestimmte Zeiträume verhindert wird oder eine bestimmte Betriebsart vorgegeben wird, wird sichergestellt, dass kurzfristige Änderungen der Ausgangssignale eines Gebers, die beispielsweise im Falle von Lagesensoren durch einen kleinen Positionswechsel hervorgerufen wurden, nicht bereits einen Wechsel der Betriebsart zur Folge haben. Diese Toleranz gegenüber Zustandsänderungen der Geber sorgt für ein kontinuierlicheres Arbeiten der Kamera. Das Zeitelement kann dabei entweder nach einem Wechsel der Betriebsart einen weiteren Wechsel für einen festgelegten Zeitraum sperren. Es kann aber auch derart eingerichtet sein, dass ein Wechsel der Betriebsart erst hervorgerufen wird, wenn das Ausgangssignal eines Gebers über einen festgelegten Zeitraum innerhalb eines bestimmten Bereichs lag. Analoges gilt für die Vorgabe einer Betriebsart.

Bei einer Weiterbildung der Erfindung ist vorgesehen, dass die Kamera ein Zeitglied umfasst, welches eine vorgegebene Betriebsart einstellt, wenn über einen vorgegebenen Zeitraum keine oder keine über einem Toleranzfenster liegende Änderung der Ausgangssignale eines oder mehrerer der Geber oder des Bildwandlers erfolgte.

Die vorgegebene Betriebsart kann beispielsweise die Erstellung eines Standbildes umfassen. So kann der Bediener ein Standbild erzeugen, indem er für einen vorgegebenen Zeitraum, beispielsweise zwischen 2 und 5 Sekunden, die Platzierung der Kamera nicht verändert.

Bei einer Weiterbildung der Erfindung ist vorgesehen, dass die Kamera ein Zeitelement umfasst, welches ein Kameraabschaltsignal abgibt, wenn längere Zeit keine oder keine über einem Toleranzfenster liegende Änderung der Ausgangssignale eines oder mehrerer der Geber oder des Bildwandlers erfolgte.

Dadurch kann die Kamera, wenn sie über einen gewissen Zeitraum vom Bediener nicht verwendet wurde, in einen Wartezustand (Stand-by) wechseln und/oder sich abschalten. Umgekehrt kann die Kamera, sobald einer der Geber anspricht, in den Betriebszustand wechseln und beispielsweise eine Auswerteeinheit und/oder ein Anzeigegerät aktivieren.

Bei einer Weiterbildung der Erfindung ist vorgesehen, dass eines der einstellbaren Elemente ein Linsenhalter, ein Bildwandler-Träger oder eine steuerbare Flüssiglinse ist.

Damit wird ermöglicht, den Fokus oder die Vergrößerung der Kamera zu beeinflussen.

Bei einer Weiterbildung der Erfindung ist vorgesehen, dass eines der einstellbaren Elemente eine Blende ist.

Dadurch wird ermöglicht, die Tiefenschärfe und die auf den Bildwandler fallende Lichtmenge einzustellen.

Bei einer Weiterbildung der Erfindung ist vorgesehen, dass eines der einstellbaren Elemente eine Beleuchtungseinrichtung ist.

Dadurch kann die Beleuchtung des abzubildenden Objekts verändert werden. Insbesondere können unterschiedliche Farben oder UV-Licht zum Einsatz kommen, was beispielsweise eine Fluoreszenzdiagnose ermöglicht.

Bei einer Weiterbildung der Erfindung ist vorgesehen, dass eine der Servoeinstelleinrichtungen ein Elektromagnet oder ein elektrischer Linearmotor ist.

Ein Elektromagnet stellt einen einfachen, kompakten und verschleißarmen Linearmotor dar, der sich zur Lageänderung einer optischen Komponente oder eines Bildwandlers gut eignet. Je nach Anforderung an die jeweilige Servoeinstelleinrichtung kann anstelle eines Elektromagneten mit zwei Endpositionen auch ein kontinuierlich einstellbarer Linearmotor verwendet werden. Außerdem kann der Elektromagnet auch mittels einer Feder in einer der Endpositionen vorgespannt sein.

Bei einer Weiterbildung der Erfindung ist vorgesehen, dass eine der Servoeinstelleinrichtungen einen Gewindetrieb und einen Elektromotor umfasst.

Mit einem Gewindetrieb, der eine Gewindespindel und eine Spindelmutter oder ein Antriebszahnrad umfasst, können präzise Translationsbewegungen über einen großen Längenbereich erreicht werden.

Bei einer Weiterbildung der Erfindung ist vorgesehen, dass mehrere Servoeinstelleinrichtungen in einer mechanischen Serienschaltung wirken.

Dadurch können Servoeinstelleinrichtungen unterschiedlichen Typs miteinander kombiniert werden und beispielsweise grobund feinschrittige Servoeinstelleinrichtungen kombiniert werden, um einerseits einen großen Bewegungsbereich und andererseits eine feine Einstellung zu ermöglichen. Auch kann beispielsweise eine grobe Servoeinstelleinrichtung dazu verwendet werden, im Wesentlichen zwischen unterschiedlichen Betriebsarten umzuschalten und über eine in Serie geschaltete zweite Servoeinstelleinrichtung Feinkorrekturen der einstellbaren Elemente vorzunehmen.

Bei einer Weiterbildung der Erfindung ist vorgesehen, dass eine der Servoeinstelleinrichtungen ein Kurvengetriebe umfasst.

Ein Kurvengetriebe, welches z.B. eine Steuerplatte miteiner Steuerkurve umfasst, erlaubt nicht-lineare Reaktionen des Abtriebteils auf das Eingangssignal zu linearisieren oder umgekehrt nicht-lineare Anworten auf das Eingangssignal gemäß Wunsch zu realisieren. Dadurch vereinfacht sich die Ansteuerung der Servoeinstelleinrichtung. Insbesondere bei der Verstellung der Blendenöffnung und bei der Einstellung des Vergrößerungsfaktors der Kamera, bei der häufig mehrere optische Elemente, wie Linsen und Bildwandler, in einem bestimmten Verhältnis zueinander verschoben werden müssen, ist ein Kurvengetriebe von Vorteil.

Gemäß weiterer vorteilhafter Ausgestaltung wird das Ausgangssignal eines der Geber, vorzugsweise eines Abstandsgebers fenstermäßig diskriminiert, wodurch ein Fenster-Geberausgangssignal erhalten wird.

Hierdurch wird erreicht, dass nicht jede kleine Änderung des Geberausgangssignales zu einer Reaktion der Servoeinstelleinrichtung führt. Dies ist in vielen Anwendungsfällen für ein ruhiges Arbeiten mit der Kamera von Vorteil.

Eine weitere vorteilhafte Fortbildung der Erfindung ist dadurch gekennzeichnet, dass aus dem Fenster-Geberausgangssignal ein Sollwertsignal für die Servoeinstellung abgeleitet wird.

Auf diese weise erhält man mit geringem apparativem Aufwand eine direkte und zuverlässige Ansteuerung der Servoeinstelleinrichtung.

Nachfolgend wird die Erfindung anhand von Ausführungsbeispielen unter Bezugnahme auf die Zeichnung erläutert. In diesen zeigen:
- Figur 1: einen vereinfachten axialen Schnitt durch eine Dentalkamera;
- Figur 2: eine teilweise weggebrochene Seitenansicht der Dentalkamera nach Figur 1;
- Figur 3: eine Dentalkamera mit Betriebseinheit, Monitor und einer kabelgetragenen Eingabeeinheit, die von Hand betätigt wird;
- Figur 4: eine Dentalkamera mit Betriebseinheit, Monitor und einem Fußschalter;
- Figur 5: einen Schnitt durch eine weitere Dentalkamera, bei der zwei mechanisch in Serie geschaltete Elektromagnete zur Verstellung des Bildwandlers verwendet werden und die eine steuerbare Linse aufweist;
- Figur 6: einen Schnitt durch eine weitere Dentalkamera, bei der ein Elektromotor mit einem Gewindetrieb zur Verstellung des Bildwandlers und ein auf eine Steuerplatte wirkender Elektromagnet zur Einstellung einer Blendenöffnung verwendet werden;
- Figur 7: einen Schnitt durch noch eine weitere Dentalkamera, bei der ein Servomotor mit einem Kurbeltrieb zur Verstellung des Bildwandlers und ein Elektromotor mit einem Gewindetrieb zur Verschiebung einer Linse verwendet werden; und
- Figur 8: eine schematische Darstellung eines Abstandsgebers für eine dentale Kamera in Verbindungmit einer Auswerte- und Steuerschaltung, die einen Servomotor stufenweise gemäß dem Sensorausgangssignal ansteuert.

Figur 1 zeigt eine Dentalkamera 10 mit einem länglichen Gehäuse 12 mit einer Längsachse 13, das sich zu seinem vorderen, in der Zeichnung oben liegenden, Ende 14 hin verjüngt.

Das um 90° abgewinkelte, vordere Ende 14 trägt in einer in der Zeichnung links gelegenen ebenen Wand ein planes transparentes Eintrittsfenster 16, das Rechteckform hat und parallel zur Längsachse 13 der Dentalkamera 10 ausgerichtet ist. Durch dieses Eintrittsfenster 16 kann von abzubildenden Zähnen 18 und 20 kommendes Licht in das Innere 22 des Gehäuses 12 einfallen.

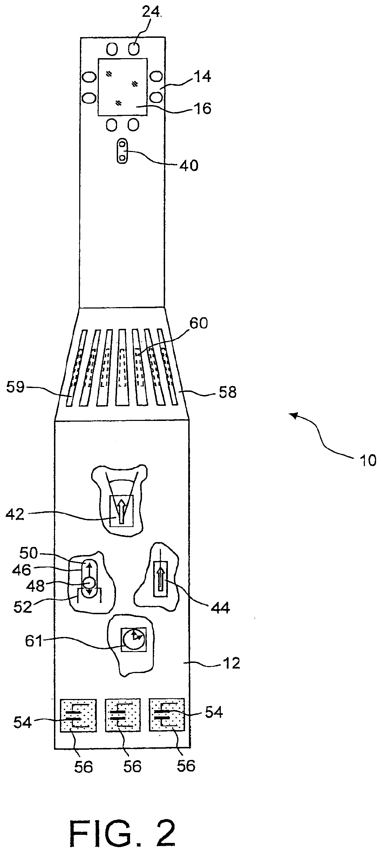

Zur Beleuchtung der Zähne 18 und 20 sind um das Eintrittsfenster 16 herum acht Leuchtdioden 24 paarweise gegenüberliegend um das Eintrittsfenster 16 herum angeordnet (vgl. Figur 2), die leicht schräg nach innen zur Fensterachse geneigt in das Gehäuse 12 eingelassen sind, so dass ein Hauptteil des von ihnen abgestrahlten Lichts von den Zähnen 18 und 20 auf das Eintrittsfenster 16 zurückgeworfen wird. Von den Leuchtdioden 24 eines Paares gibt jeweils eine Weißlicht, die andere farbiges Licht oder auch UV-Licht ab. Die Leuchtdioden 24 können einzeln angesteuert und in ihrer Helligkeit variiert werden.

Im Inneren 22 des Gehäuses 12 befindet sich hinter dem Eintrittsfenster 16 ein unter 45° zur Längsachse 13 geneigter Spiegel 26. Der Spiegel 26 wirft das einfallende Licht über eine symbolisch durch drei Linsengruppen L1, L2 und L3 verkörperte Optik entlang der Längsachse 13 auf einen Bildwandler 28, der auf einer Trägerplatte 30 angeordneten ist.

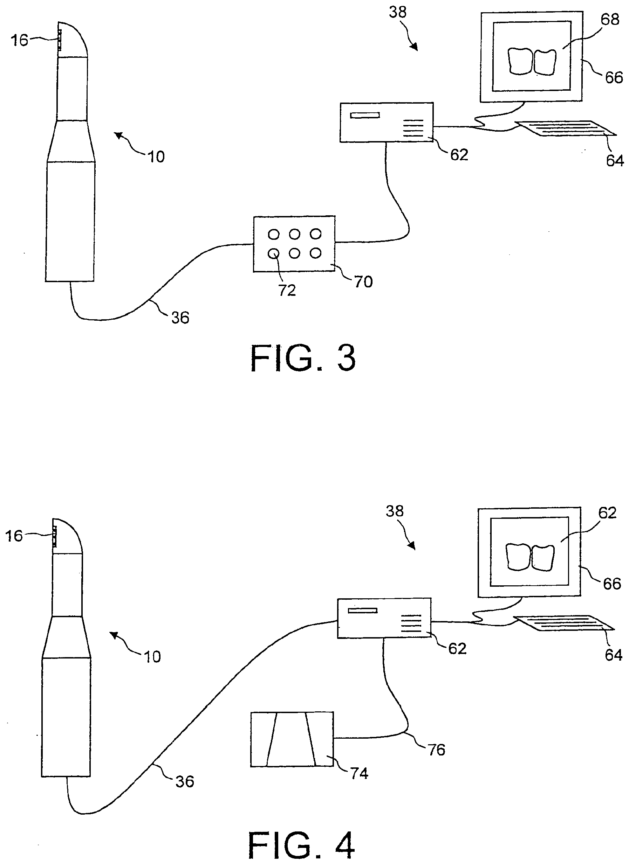

Der Bildwandler 28 ist über eine elektrische Verbindung 32 mit einer Kameraelektronik 34 verbunden, welche die vom Bildwandler 28 bereitgestellten Signale ausliest, verstärkt und über ein Betriebskabel 36 an eine in den Figuren 3 und 4 gezeigte Betriebseinheit 38 übermittelt. Die Kameraelektronik 34 stellt auch die vom Bildwandler 28 benötigten Versorgungsspannungen bereit.

Zur Bestimmung der Gegenstandsweite umfasst die Dentalkamera 10 einen nahe dem Eintrittsfenster 16 angeordneten IR-Abstandssensor 40, der über eine IR-LED 40a zur Abstandsmessung IR-Lichtpulse aussendet und das reflektierte IR-Licht mit einem Fototransistor 40b erfasst. Z.B. über die Laufzeit wird so der Abstand des Eintrittsfensters 16 zu den Zähnen 18 und 20 und damit auch der optische Abstand zwischen den Zähnen 18 und 20 und der Optik, d.h. den Linsengruppen L1, L2 und L3 und dem Bildwandler 28 ermittelt.

In einer intraoralen Betriebsart, bei der das vordere Ende 14 der Dentalkamera 10 in das Mundinnere eingebracht wird, um die einzelnen Zähne abzubilden, ist dieser Abstand im Allgemeinen gering, sodass die Optik in einen Makromodus umgestellt werden muss. In einer extraoralen Betriebsart, bei der die Dentalkamera, 10 vor das Gesicht eines Patienten gehalten wird, um den vorderen Teil des Zahnbogens abzubilden, ist dieser Abstand hingegen größer und die Optik muss auf eine größere Gegenstandsweite eingestellt werden, um ein scharfes Bild zu erhalten.

Figur 2 zeigt in einer teilweise weggebrochene Seitenansicht alternative Geber der Dentalkamera 10, die je nach Ausführungsbeispiel in Kombination oder einzeln zur Einstellung verschiedener Betriebsarten der Dentalkamera 10 dienen.

Die Dentalkamera 10 umfasst einen Neigungssensor 42, der so angeordnet ist, dass er die Neigung der Längsachse 13 der Dentalkamera 10 zur Vertikalen parallel zur Ebene des Eintrittsfensters 16 misst. Ein weiterer Neigungssensor 44 ist so angeordnet, dass er die Neigung der Längsachse 13 senkrecht zur Ebene des Eintrittsfensters 16 erfasst.

Ein einfacher Neigungssensor 46, bei dem eine kleine Kugel 48 aus leitfähigem Material frei verschiebbar in einem länglichen Hohlraum 50 angeordnet ist, ist so angeordnet, dass die Kugel 48 aufgrund der Schwerkraft einen elektrischen Kontakt 52 schließt, wenn das vordere Ende 14 der Dentalkamera, 10 nach oben weist.

Nahe ihrem hinteren Ende weist die Dentalkamera 10 mehrere über den Gehäuseumfang verteilte kapazitive Sensoren 54 auf. Die kapazitiven Senscren sind dabei im Inneren 22 des Gehäuses 12 hinter Gehäusebereichen 56 mit verminderter Wandstärke angeordnet. Durch die Anordnung am hinteren Ende der Dentalkamera 10 ermöglichen die kapazitiven Sensoren 54 zu erkennen, ob und wo die Dentalkamera 10 von einem Bediener am hinteren Ende umfasst wird.

An einem sich zum vorderen Ende 14 hin konisch verjüngenden Griffbereich 58, an dem die Dentalkamera 10 zur Positionierung im intraoralen Mundbereich erfasst wird, sind mehrere längliche Gummielemente 59 angeordnet, die in Längsrichtung verlaufen und um den Umfang des Gehäuses 12 verteilt sind. Die Gummielumente 59 sind Ausstülpungen einer Gummimanschette, die auf ihrer Innenseite mehrere Dehnungsmessstreifen 60 trägt. Bei einem bestimmten Druck auf eines oder mehrere der Gummielemente 59 geben die Dehnungsmessstreifen 60 ein Signal ab. Ähnlich den kapazitiven Sensoren 54 kann mit dieser Art manuell betätigtem Geber erkannt werden, ob und wo die Dentalkamera 10 im Griffbereich 58 umfasst wird.

Die Dentalkamera 10 weist ferner ein Zeitglied 61 auf, das mit der Kameraelektronik 34 verbunden oder Teil dieser ist. Das Zeitglied 61 dient dazu, ein rasches Nachsteuern der Kameraeigenschaften bei transienten Störeinflüssen auf die Signale der verschiedenen Geber zu vermeiden.

Hierzu kann das Zeitglied in einem Steuerkanal für eine Servostelleinrichtung eingefügt sein, wodurch alle transienten Störsignale aller Geber für diese Servostelleinrichtung ohne Einfluss bleiben. Das Zeitglied kann aber auch in den einem Geber nachgeschalteten Signalkanal integriert sein, so dass nur kurzfristige Signaländerungen dieses Gebers unberücksichtigt bleiben, diejenigen anderer Geber dagegen berücksichtigt werden.

Bei beiden Varianten kann man mehrere auf unterschiedliche Zeiten eingestellte Zeitglieder verwenden, die z.B. die Ausgangssignale verschiedener Geber auf Länge ihres Vorliegens prüfen.

Ist ein Zeitglied einem Geber nachgeschaltet, führt die Kameraelektronik 34 eine Änderung aufgrund eines geänderten Ausgangssignals eines Gebers erst durch, wenn das geänderte Ausgangssignal des Gebers über einen von dem Zeitglied 61 festgelegten Zeitraum hinaus anlag.

Bei einem anderen Ausführungsbeispiel oder für einen anderen Geber verhindert die Kameraelektronik 34 mit Hilfe des Zeitglieds 61 nach einer Änderung der Abbildungseigenschaften oder einem Wechsel der Betriebsart für einen festgelegten Zeitraum weitere Änderungen oder Wechsel.

Ferner kann die Kameraelektronik 34 mit Hilfe des Zeitglieds 61 bestimmen, wie lange die Ausgangssignale eines oder mehrerer Geber keine Änderungen oberhalb einer festlegbaren Schwelle mehr zeigten. Dies ist beispielsweise der Fall, wenn der Bediener die Position der Dentalkamera 10 für einen bestimmten Zeitraum beibehält. Die Kameraelektronik 34 löst daraufhin die Aufnahme eines Standbildes in einem Aufnahmemodus aus. In diesem Aufnahmemodus wird unter anderem die Belichtungszeit des Bildwandlers 28 verkürzt und die Beleuchtung mit Hilfe der Leuchtdioden 24 entsprechend erhöht, damit ein möglichst verwackelungsfreies Bild entsteht. Bewegt der Bediener die Dentalkamera, 10 wieder, schalter die Kameraelektronik 34 in den Videomodus zurück.

Liegt über einen längeren Zeitraum, der wiederum mittels des Zeitglieds 61 bestimmt wird keine Änderung der Ausgangssignale vor, so sendet die Dentalkamera 10 ein Abschaltsignal an die Betriebseinheit 38.

Wie die Figuren 3 und 4 zeigen, ist die Dentalkamera 10 über das Betriebskabel 36 mit der Betriebseinheit 38 verbunden, die für die Dentalkamera, 10 die notwendigen Betriebsspannungen und Steuersignale bereitstellt und die erhaltenen Bildsignale aufbereitet, verarbeitet und speichert. Die Betriebseinheit 38, die einen geeignet programmierten Rechner 62 umfassen kann, ist ferner mit einem Tastenfeld 64 ausgestattet. Schließlich weist sie einem Monitor 66 auf, der so ein Bild 68 der Zähne 18 und 2C zeigt.

Im Ausführungsbeispiel der Figur 3 ist zwischen der Dentalkamera 10 und der Betriebseinheit 38 ein kabelgetragenes Bedienpanel 70 mit verschiedenen manuellen Bedienelementen 72 angeordnet. Das Bedienpanel 70 überträgt über das Betriabskabel 36 Informationen zur Änderung von Kameraparametern wie Fokus, Beleuchtung, Tiefenschärfe und/oder sonstige Farb-, Kontrast- oder Weißabgleiche zu der Dentalkamera 10 und/oder der Betriebseinheit 38 und erlaubt zusätzliche manuelle Eingaben zur Vorgabe oder Änderung von Kameraparametern und zum Eingriff in die Kamerasteuerung.

Im Ausführungsbeispiel der Figur 4 wird anstatt des Bedienpanels 70 ein kabelgetragener Fußschalter 74 verwendet, der über eine Steuerleitung 76 an die Betriebseinheit 38 angeschlossen ist, um die Bildeigenschaften des Monitors 66 oder die Abbildungseigenschaften der Dentalkamera 10 zu ändern.

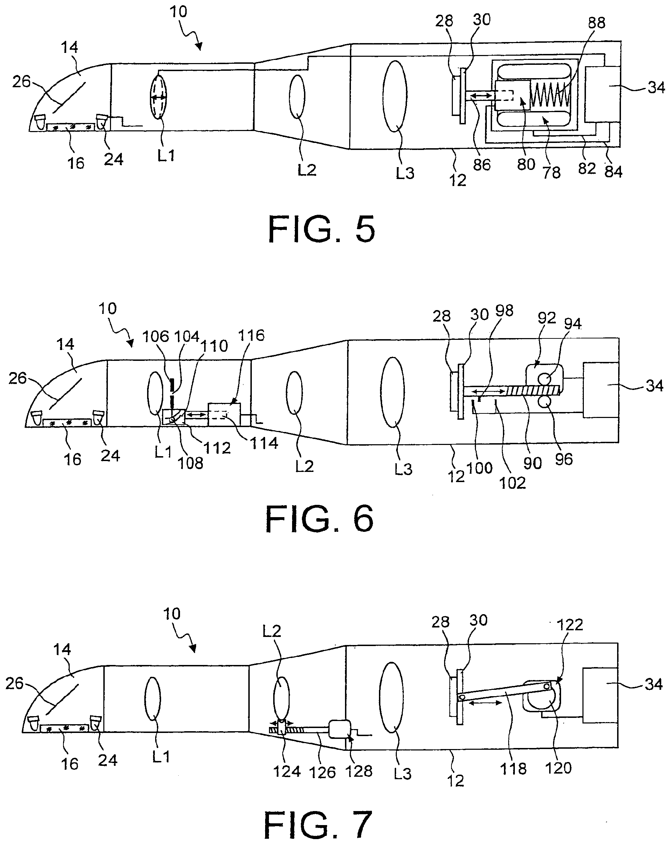

Figur 5 zeigt einen Längsschnitt durch ein Ausführungsbeispiel der Dentalkamera 10, bei der die Linsengruppe L1 eine elektrisch steuerbare Flüssiglinse umfasst, die mit der Kameraelektronik 34 verbunden ist. So kann die Brennweite der Linsengruppe L1 geändert werden.

Ferner weist die Dentalkamera 10 zur Verschiebung des Bildwandlers 28 samt Trägerplatte 30 einen äußeren Elektromagneten 78 und einen inneren Linearmotor 80 auf, die in Serie geschaltete Linearaktoren darstellen und über die Leitungen 82 und 84 mit der Kameraelektronik 34 verbunden sind. Der Anker des äußeren Elektromagneten ist dabei zugleich das Gehäuse des Linearmotors 80.

Der Linearmotor 80 steuert kontinuierlich die Translationsbewegung einer in ihm laufenden zylindrischen Stange 86, an deren vorderen Ende die Trägerplatte 30 befestigt ist. Der Linearmotor 80 wird dabei im verglichen mit ihm deutlich längeren Elektromagnet 78 über eine Feder 88 in eine vordere Endposition vorgespannt. Mit Hilfe des Elektromagneten 78, bei dem nur zwischen einem eingeschalteten und einem abgeschalteten Zustand unterschieden werden kann, kann somit die Lage des Linearmotors 80 in der einen oder der anderen Endposition festgelegt werden. Damit kann mit dem Elektromagneten 78 grob zwischen einer Lage des Bildwandlers 28 in einem Makromodus und einer Einstellung auf Unendlich umgeschaltet werden, und der Linearmotor 80 kann zur Feineinstellung des Fokus verwendet werden.

Wie weiter durch den Anfang eine Verbindungleitung an den Leuchtdioden 24 angedeutet ist, können über die Kameraelektronik 34 die Leuchtstärken der Leuchtdioden 24 gesteuert bzw. die Leuchtdioden 24 einer bestimmten Farbe an-oder abgeschaltet werden.

Figur 6 zeigt einen Längsschnitt durch ein weiteres Ausführungsbeispiel einer Dentalkamera 10, bei der der Bildwandler 28 über einen Gewindetrieb verstellt wird. Hierzu ist an der Trägerplatte 30 eine Gewindestange 90 angebracht, in welche ein mit einem Elektromotor 92 verbundenes Schneckenzahnrad 94 eingreift. Als Gegenhalterung ist dem Schneckenzahnrad 94 bezüglich der Gewindestange 90 gegenüberliegend eine Stützrolle 96 angeordnet.

Ferner trägt die Gewindestange 90 eine Fahne 98, die zwei Endkontakte 100 und 102 betätigen kann.

Zur Steuerung einer Blendenöffnung 104 ist an einer Blende 106 ein Blendenstellglied 108 angebracht, dessen Betätigungsende längs einer Steuerkurve 110 läuft, die durch zwei Führungsrippen begrenzt ist, die auf einer Steuerplatte 112 aufgebracht sind und insgesamt in einem Winkel von etwa 45° zum Blendenstellglied 108 verlaufen.

Wird die Steuerplatte 104 über eine zylindrische Stange 114, die von einem elektrischen Linearmotor 116 bewegt wird, verschoben, folgt das Blendenstellglied 108 der Streuerkurve 110. Durch die Geometrie der Steuerkurve 110 lässt sich auf einfache Weise eine nicht lineares Öffnungsverhalten der Blende 106 erreichen.

Figur 7 zeigt ein weiteres Ausführungsbeispiel einer Dentalkamera 10, bei dem der Bildwandler 28 über eine Pleuelstange 118, die mit der Trägerplatte 30 des Bildwandlers 28 und einem Exzenter 120 jeweils gelenkig verbunden ist, hin-und herbewegt wird. Der Exzenter 120 wird dabei von einem Servomotor 122 angetrieben, der durch die Kameraelektronik 34 gesteuert wird.

Weiter zeigt Figur 7 eine Linsenhalterung 124 der Linsengruppe L2, die über einen Gewindetrieb verschiebbar ist. Der Gewindetrieb umfasst dazu eine Gewindestange 126, die direkt von einem Elektromotor 128 angetrieben wird und in einer Gewindebohrung der Linsenhalterung 124 läuft. Durch Erregen des Elektromotors 128 wird die Linsenhalterung 124 gesteuert durch die Kameraelektronik 34 verschoben.

Die oben beschriebenen Dentalkameras 10 funktionieren derart, dass mit den verschiedenen Gebern beispielsweise durch Erfassung der Kameralage mit Hilfe der Neigungssensoren 42, 44 und 46 oder durch den IR-Sensor 40 bestimmte Betriebsarten wie beispielsweise eine intraorale und extraorale Betriebsart der Dentalkamera 10 erkannt werden und dann mit Hilfe der Kameraelektronik 34 und mittels Servoantrieben an den verschiedenen steuerbaren Elementen (darunter elektromechanische und elektrische/elektrische sowie Software-Elemente) entsprechende Anpassungen der Kameraparameter vorgenommen werden.

Andere Geber wie die kapazitiven Sensoren 54 oder die Gummielemente 60 sowie das kabelgetragene Bedienpanel 64 oder der Fußschalter 68 werden vom Bediener manuell betätigt, wodurch eine gewünschte Betriebsart bzw. eine gewünschte Änderung eines Arbeitsparameters erkannt wird und eine entsprechende Änderung der Kameraparameter erfolgt.

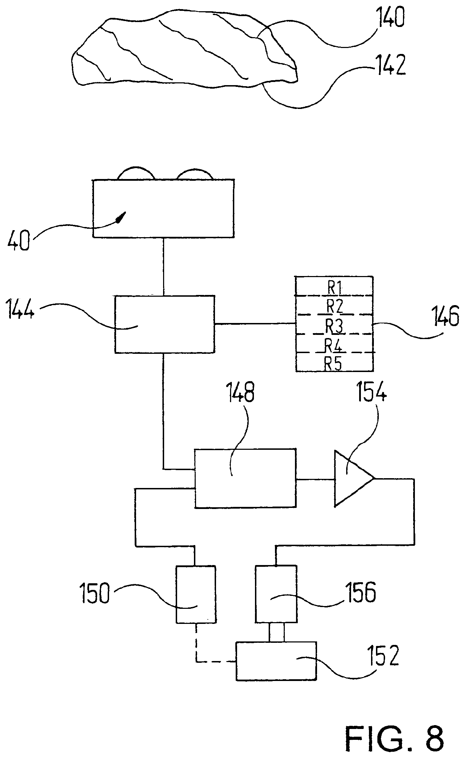

In Figur 8 ist bei 140 ein Ausschnitt eines Gewebes dargestellt, welches eine freie Oberfläche 142 aufweist. Unter Abstand vor der Oberfläche 142 ist ein Infrarot-Abstandssensor 40 angeordnet, wie er schon obenstehend beschrieben wurde. Der Infrarot-Abstandssensor 40 erzeugt ein analoges Abstandssignal, welches proportional zum Abstandssensor 40 und Oberfläche 142 ist.

Das Ausgangssignal des Abstandssensors 40 wird auf einen Rechenkreis 144 gegeben, der das analoge Ausgangssignal des Abstandssensors 40 in ein digitales Signal umsetzt und das so erhaltene digitale Ausgangssignal mit mehreren Referenzwerten R1 bis R5 vergleicht, die in einem Referenzwertspeicher 146 abgelegt sind.

Der Rechenkreis 144 gibt 6 verschiede Sollwertsignale an seinem Ausgang ab, je nachdem, ob das erhaltene Abstandsignal kleiner ist als R1, zwischen R1 und R2, zwischen R2 und R3, zwischen R3 und R4, zwischen R4 und R5 oder größer als R5 ist.

Das jeweils errechnete Sollwertsignal wird auf einen Eingang eines Fehlerkreises 148 gegeben, der an seinem zweiten Eingang das Ausgangssignal eines Stellungsmessers 150 erhält, der mechanisch mit einer zu bewegenden Last 152 gekoppelt ist. Bei der Last kann es sich z.B. um den Schlitten 30 für den Bildwandler 28 handeln. Das vom Ausgang des Fehlerkreises 148 bereitgestellte Fehlersignal wird über einen Verstärker 154 auf die Steuerklemme eines Linearmotors 156 gegeben, der die Last 152 antreibt. Der Linearmotor 156 kann direkt ein magnetischer Linearmotor sein; es kann sich hierbei aber auch um die Kombination eines Drehantriebes mit einem Getriebe zum Umsetzen der Drehbewegung des Antriebes in eine Linearbewegung (z.B. Zahnstangentrieb)handeln.

Die in Figur 8 gezeigte und obenstehend beschriebene Servo-Fokussierung für eine dentale Kamera arbeitet folgendermaßen :
Der Abstandssensor 40 ermittelt den momentanen Abstand der Kamera von der Oberfläche 142. Der Rechenkreis 144 ermittelt, welcher Entfernungsstufe der gerade gemessene Abstand entspricht und erzeugt ausgehend von der so berechneten Stufe ein Sollwertsignal für den Linearmotor 156. Die den Fehlerkreis 148 und den Stellungsmesser 150 umfassende Regelschleife stellt dann die Last 152 so, wie dies zur scharfen Abbildung der Oberfläche 142 auf dem Bildwandler 28 notwendig ist.

Folgende Abwandlungen der Erfindung sind mit zu berücksichtigen:
Eine der Servoeinstelleinrichtungen umfasst einen Elektromagnet oder einen elektrischen Linearmotor.
Eine der Servoeinstelleinrichtungen umfasst einen Gewindetrieb und einen Elektromotor.
Eine der Servoeinstelleinrichtungen umfasst ein Kurvengetriebe.

## Patentansprüche

1. Dentale oder medizinische Kamera (10) mit einer Mehrzahl von Komponenten, darunter eine Optik (L1; L2; L3) und ein Bildwandler (28), wobei die Komponenten mindestens ein einstellbares Element (L1; L2; 24; 28; 30; 106; 124) umfassen; bei der für mindestens eines der einstellbaren Elemente (L1; L2; 24; 28; 106; 124) mindestens eine Servoeinstelleinrichtung (34; 78; 80; 92; 116; 122; 128) vorgesehen ist und die mindestens einen Geber (40; 42; 44; 46; 54; 60; 64; 70; 74) aufweist, durch den mindestens zwei verschiedene Betriebsarten der Kamera (10) unterscheidbar sind und dessen Ausgangssignal bei der Ansteuerung mildestens einer der Servoeinstelleinrichtungen (34; 78; 80; 92; 116; 122; 128) verwendet wird, **dadurch gekennzeichnet, dass** die Kamera ein Zeitglied (61) umfasst, welches während eines festgelegten Zeitraums einen Wechsel der Betriebsart verhindert.

2. Kamera nach Anspruch 1, **dadurch gekennzeichnet, dass**
die Kamera ein Zeitglied (61) umfasst, welches eine vorgegebene Betriebsart einstellt, wenn über einen vorgegebenen Zeitraum keine oder keine über einen Toleranzfenster liegende Änderung der Ausgangssignale eines oder mehrerer der Geber (40; 42; 44; 46; 54; 60; 64; 10; 74) oder des Bildwandlers (28) erfolgte.

3. Kamera nach Anspruch 1 oder 2, **dadurch gekennzeichnet,**
**dass** sie ein Zeitglied (61) umfasst, welches ein Kameraabschaltsignal abgibt, wenn in einer vorgegebenen Zeitspanne keine oder keine über einem Toleranzfenster liegende Änderung der Ausgangssignale eines oder mehrerer der Geber (40; 42; 44; 46; 54; 60; 64; 70; 74) oder des Bildwandlers (28) erfolgte.

4. Kamera nach einem der Ansprüche, 1 bis 3, **dadurch gekennzeichnet, dass** unter den Gebern (40; 42; 44; 46; 54; 60; 64; 70; 74) ein Bewegungs- oder Abstandssensors (40), vorzugsweise ein optischer Bewegungs- oder Abstandsensor, ist.

5. Kamera nach Anspruch 4, dadurch gekennzeichet, dass
der Bewegungs- oder Abstandssensor (40) nahe einem Eintrittsfenster (16) der Kamera (10) angeordnet ist.

6. Kamera nach einem der Ansprüche 1 bis 5, **dadurch gekennzeichnet, dass** unter den Gebern (40; 42; 44; 46; 54; 60; 64; 70; 74) ein Sensor ist, der aus der nachfolgenden Gruppe ausgewählt ist: ein Lagesensor (42; 44; 46), insbesondere ein Neigungssensor oder eine Neigungsschaltereinheit, ein Beschleunigungssensor, insbesondere ein Vibrationssensor, ein kapazitiver Sensor (54), ein optischer Sensor, ein magnetischer Sensor oder ein vorzugsweise als Piezosensor ausgebildeter Drucksensor (60).

7. Kamera nach einem der Ansprüche 1 bis 6, **dadurch gekennzeichnet, dass** unter den Gebern (40; 42; 44; 46; 54; 60; 64; 70; 74) ein manuell betätigter Geber (54; 60; 64; 70; 74) ist.

8. Kamera nach Anspruch 7, **dadurch gekennzeichnet, dass**
der manuell betätigte Geber (54; 60; 64; 70; 74) ein kapazitiver Sensor (54), ein optischer Sensor oder ein Drucksensor (60), insbesondere ein Piezosensor, ist.

9. Kamera nach Anspruch 7 oder 8, **dadurch gekennzeichnet,**
**dass** der manuell betätigte Geber (54; 60; 64; 70; 74) Teil einer Eingabeeinheit ist, z.B. einer Handeingabeeinheit (70), einer Fußeingabeeinheit (74) oder eines Bedienpanels (64) einer Kamerabetriebseinheit (38).

10. Kamera nach einem der Ansprüche 1 bis 9, **dadurch gekennzeichnet, dass** unter den Gebern (40; 42; 44; 46; 54; 60; 64; 70; 74) eine Sprachsteusrung ist.

11. Kamera nach einem der vorherigen Ansprüche, **dadurch**
**gekennzeichnet, dass** eines der einstellbaren Elemente (L1; L2; 24; 28; 30; 106; 124) ein Linsenhalter (124), ein Bildwandler-Träger (30) oder eine steuerbare Flüssiglinse (L1) ist.

12. Kamera nach einem der vorherigen Ansprüche, **dadurch**
**gekennzeichnet, dass** eines der einstellbaren Elemente (L1; L2; 24; 28; 30; 106; 124) eine Blende (106) ist.

13. Kamera nach einem der vorherigen Ansprüche, **dadurch**
**gekennzeichnet, dass** eines der einstellbaren Elemente (L1; L2; 24; 28; 30; 106; 124) eine Beleuchtungseinrichtung (24) ist.

14. Kamera nach einem der vorherigen Ansprüche, **dadurch**
**gekennzeichnet, dass** mehrere Servoeinstelleinrichtungen (34; 78; 80; 92; 116; 122; 128) in Reihenschaltung wirken.

15. Kamera nach einem der Ansprüche 1 bis 14, **dadurch**
**gekennzeichnet, dass** das Ausgangssignal mindestens eines der Geber, vorzugsweise eines Abstandsgebers (40) fenstermäßig diskriminiert wird, wodurch ein Fenster-Geberausgangssignal erhalten wird.

16. Kamera nach Anspruch 15, **dadurch gekennzeichnet,**
**dass** aus dem Fenster-Geberausgangssignal ein Sollwersignal für die Servoeinstelleinrichtung (144, 150, 156) abgeleitet wird.

## Claims

1. Dental or medical camera (10) comprising a plurality of components, including an optical unit (L1; L2; L3) and an image converter (28), the components comprising at least one adjustable element (L1; L2; 24; 28; 30; 106; 124); in which provision is made of at least one servo-adjustment device (34; 78; 80; 92; 116; 122; 128) for at least one of the adjustable elements (L1; L2; 24; 28; 106; 124) and which has at least one transducer (40; 42; 44; 46; 54; 60; 64; 70; 74), by means of which at least two different modes of operation of the camera (10) are distinguishable and the output signal of which is used in the actuation of at least one of the servo-adjustment devices (34; 78; 80; 92; 116; 122; 128), **characterized in that** the camera comprises a timer (61) which prevents a change in the mode of operation during a predetermined period of time.

2. Camera according to Claim 1, **characterized in that** the camera comprises a timer (61) which sets a predetermined mode of operation if, over a predetermined period of time, there was no change, or no change lying over a tolerance window, of the output signals of one or more of the transducers (40; 42; 44; 46; 54; 60; 64; 70; 74) or of the image converter (28).

3. Camera according to Claim 1 or 2, **characterized in that** it comprises a timer (61) which emits a camera switch-off signal if, over a predetermined time interval, there was no change, or no change lying over a tolerance window, of the output signals of one or more of the transducers (40; 42; 44; 46; 54; 60; 64; 70; 74) or of the image converter (28).

4. Camera according to one of Claims 1 to 3, **characterized in that** the transducers (40; 42; 44; 46; 54; 60; 64; 70; 74) include a movement or distance sensor (40), preferably an optical movement or distance sensor.

5. Camera according to Claim 4, **characterized in that** the movement or distance sensor (40) is arranged near an entrance window (16) of the camera (1).

6. Camera according to one of Claims 2 to 5, **characterized in that** the transducers (40; 42; 44; 46; 54; 60; 64; 70; 74) include a sensor selected from the following group: a position sensor (42; 44; 46), in particular an inclination sensor or an inclination switch unit, an acceleration sensor, in particular a vibration sensor, a capacitive sensor (54), an optical sensor, a magnetic sensor or a pressure sensor (60) preferably embodied as a piezo-sensor.

7. Camera according to one of Claims 1 to 6, **characterized in that** the transducers (40; 42; 44; 46; 54; 60; 64; 70; 74) include a manually actuated transducer (54; 60; 64; 70; 74).

8. Camera according to Claim 7, **characterized in that** the manually actuated transducer (54; 60; 64; 70; 74) is a capacitive sensor (54), an optical sensor or a pressure sensor (60), in particular a piezo-sensor.

9. Camera according to Claim 7 or 8, **characterized in that** the manually actuated transducer (54; 60; 64; 70; 74) is part of an input unit, e.g. of a hand input unit (70), a foot input unit (74) or an operating panel (64) of a camera operating unit (38).

10. Camera according to one of Claims 1 to 9, **characterized in that** the transducers (40; 42; 44; 46; 54; 60; 64; 70; 74) include a voice control.

11. Camera according to one of the preceding claims, **characterized in that** a lens holder (124), an image-converter support (30) or a controllable liquid lens (L1) is one of the adjustable elements (L1; L2; 24; 28; 30; 106; 124).

12. Camera according to one of the preceding claims, **characterized in that** a stop (106) is one of the adjustable elements (L1; L2; 24; 28; 30; 106; 124).

13. Camera according to one of the preceding claims, **characterized in that** an illumination device (24) is one of the adjustable elements (L1; L2; 24; 28; 30; 106; 124).

14. Camera according to one of the preceding claims, **characterized in that** a plurality of servo-adjustment devices (34; 78; 80; 92; 116; 122; 128) act in a series connection.

15. Camera according to one of Claims 1 to 14, **characterized in that** the output signal of at least one transducer, preferably of a distance transducer (40), is discriminated in a window-type manner, as a result of which a window transducer output signal is obtained.

16. Camera according to Claim 15, **characterized in that** an intended value signal for the servo-adjustment device (144, 150, 156) is derived from the window transducer output signal.

## Revendications

1. Caméra dentaire ou médicale (10) avec une pluralité de composants, parmi lesquels une optique (L1; L2 ; L3) et un convertisseur d'image (28), sachant que les composants comprennent au moins un élément réglable (L1 ; L2 ; 24 ; 28 ; 30 ; 106 ; 124) ; selon laquelle il est prévu, pour au moins un des éléments réglables ((L1 ; L2 ; 24 ; 28 ; 30 ; 106 ; 124), au moins un dispositif de servoréglage (34 ;78 ; 80 ; 92 ; 116 ; 122 ; 128) qui présente au moins un transmetteur (40 ; 42 ; 44 ; 46 ; 54 ; 60 ; 64 ; 70 ; 74), par lequel au moins deux modes de fonctionnement différents de la caméra (10) peuvent être différenciés et dont le signal de sortie est utilisé pour la commande d'au moins un des dispositifs de servoréglage (34 ;78 ; 80 ; 92 ; 116 ; 122 ; 128),
**caractérisée en ce que** la caméra comprend un organe de temporisation (61), qui empêche un changement du mode de fonctionnement pendant une durée déterminée.

2. Caméra selon la revendication 1, **caractérisée en ce que** la caméra comprend un organe de temporisation (61) qui règle un mode de fonctionnement prédéfini lorsque, pendant une durée prédéfinie, il n'y a pas eu de modification des signaux de sortie d'un ou plusieurs des transmetteurs (40 ; 42 ; 44 ; 46 ; 54 ; 60 ; 64 ; 70 ; 74) ou du convertisseur d'image (28), ou pas de modification supérieure à une fenêtre de tolérance.

3. Caméra selon la revendication 1 ou 2, **caractérisée en ce qu'**elle comprend un organe de temporisation (61) qui délivre un signal de désactivation de la caméra lorsque, pendant une durée prédéfinie, il n'y a pas eu de modification des signaux de sortie d'un ou plusieurs des transmetteurs (40 ; 42 ; 44 ; 46 ; 54 ; 60 ; 64 ; 70 ; 74) ou du convertisseur d'image (28), ou pas de modification supérieure à une fenêtre de tolérance.

4. Caméra selon l'une des revendications 1 à 3, **caractérisée en ce qu'**un capteur de déplacement ou de distance (40), de préférence un capteur de déplacement ou de distance optique, est présent parmi les transmetteurs (40 ; 42 ; 44 ; 46 ; 54 ; 60 ; 64 ; 70 ; 74).

5. Caméra selon la revendication 4, **caractérisée en ce que** le capteur de déplacement ou de distance (40) est disposé à proximité d'une fenêtre d'entrée (16) de la caméra (10).

6. Caméra selon l'une des revendications 1 à 5, **caractérisée en ce qu'**est présent parmi les transmetteurs (40 ; 42 ; 44 ; 46 ; 54 ; 60 ; 64 ; 70 ; 74) un capteur qui est sélectionné dans le groupe suivant : un capteur de position (42 ; 44 ; 46), en particulier un capteur d'inclinaison ou une unité de déclenchement par inclinaison, un capteur d'accélération, en particulier un capteur à lames vibrantes, un capteur capacitif (54), un capteur optique, un capteur magnétique ou un capteur de pression (60) réalisé de préférence sous forme de capteur piézoélectrique.

7. Caméra selon l'une des revendications 1 à 6, **caractérisée en ce qu'**un transmetteur à actionnement manuel (54 ; 60 ; 64 ; 70 ; 74) est présent parmi les transmetteurs (40 ; 42 ; 44 ; 46 ; 54 ; 60 ; 64 ; 70 ; 74).

8. Caméra selon la revendication 7, **caractérisée en ce que** le transmetteur à actionnement manuel (54 ; 60 ; 64 ; 70 ; 74) est un capteur capacitif (54), un capteur optique ou un capteur de pression (60), en particulier un capteur piézoélectrique.

9. Caméra selon la revendication 7 ou 8, **caractérisée en ce que** le transmetteur à actionnement manuel (54 ; 60 ; 64 ; 70 ; 74) fait partie d'une unité d'entrée, par exemple d'une unité d'entrée à main (70), d'une unité d'entrée au pied (74) ou d'un panneau de commande (64) d'une unité de commande de caméra (38).

10. Caméra selon l'une des revendications 1 à 9, **caractérisée en ce qu'**une commande vocale est présente parmi les transmetteurs (40 ; 42 ; 44 ; 46 ; 54 ; 60 ; 64 ; 70 ; 74).

11. Caméra selon l'une des revendications précédentes, **caractérisée en ce qu'**un des éléments réglables ((L1 ; L2 ; 24 ; 28 ; 30 ; 106 ; 124) est un porte-lentille (124), un support (30) de convertisseur d'image ou une lentille liquide asservissable (L1).

12. Caméra selon l'une des revendications précédentes, **caractérisée en ce qu'**un des éléments réglables ((L1 ; L2 ; 24 ; 28 ; 30 ; 106 ; 124) est un diaphragme (106).

13. Caméra selon l'une des revendications précédentes, **caractérisée en ce qu'**un des éléments réglables ((L1 ; L2 ; 24 ; 28 ; 30 ; 106 ; 124) est un dispositif d'éclairage (24).

14. Caméra selon l'une des revendications précédentes, **caractérisée en ce que** plusieurs dispositifs de servoréglage (34 ; 78 ; 80 ; 92 ; 116 ; 122 ; 128) agissent en montage en série.

15. Caméra selon l'une des revendications 1 à 14, **caractérisée en ce que** le signal de sortie d'au moins un des transmetteurs, de préférence d'un indicateur de distance (40), est discriminé de manière fenêtrée, de sorte qu'on obtient un signal de sortie de transmetteur fenêtré.

16. Caméra selon la revendication 15, **caractérisée en ce qu'**on déduit du signal de sortie de transmetteur fenêtré un signal de valeur de consigne pour le dispositif de servoréglage (144, 150, 156).
